# EUROPEAN PATENT APPLICATION

(11) **EP 4 696 674 A1**
(43) Date of publication of application: **18.02.2026**
(21) Application number: 24788717.7
(22) Date of filing: 09.04.2024
(51) Int. Cl.: C07C 37/20, C07C 39/15, C07C 59/68, C07C 59/70, C07C 67/31, C07C 67/343, C07C 69/675, C07C 69/712, C07C 69/734, C07C 69/736

(54) **METHOD FOR PRODUCING 3-HYDROXYBIPHENYL COMPOUND AND DERIVATIVE THEREOF**

(30) Priority: 10.04.2023 JP 2023063232; 13.11.2023 JP 2023192831
(71) Applicant: Sumitomo Chemical Company, Limited, Tokyo 103-6020 (JP)
(72) Inventor: MAEHATA, Ryota, Takarazuka-shi, Hyogo 665-8555 (JP); TANIMURA, Shun, Takarazuka-shi, Hyogo 665-8555 (JP); IGUCHI, Miyuki, Takarazuka-shi, Hyogo 665-8555 (JP); OKURA, Yuka, Takarazuka-shi, Hyogo 665-8555 (JP); SASAKURA, Kohei, Takarazuka-shi, Hyogo 665-8555 (JP)
(74) Representative: Vossius & Partner Patentanwälte Rechtsanwälte mbB
(86) International application number: PCT/JP2024/014384
(87) International publication number: WO 2024/214693

(57) **Abstract**

The present invention provides a novel method for producing a 3-hydroxybiphenyl compound, and a novel method for producing a derivative of 3-hydroxybiphenyl compound using the same. Specifically, the present invention provides a method for producing a compound represented by formula (3) 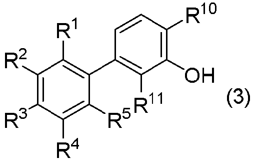 [wherein: R1, R2, R3, R4, and R5 are identical to or different from each other, and each represents a hydrogen atom or the like; and R10 and R11 are identical to or different from each other, and each represents a hydrogen atom or the like] which comprises reacting a compound represented by formula (1) 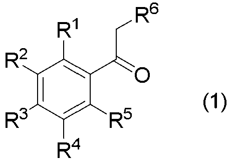 [wherein: R1, R2, R3, R4, and R5 are the same as defined above; and R6 represents a phenylsulfonyl group or the like] and a compound represented by formula (2) 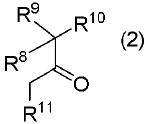 [wherein: the combination of R8 and R9 represents a combination wherein R8 represents CH2NR12R13 or the like, and R9 represents a hydrogen atom, or the like; R10 and R11 are the same as defined above; and R12 and R13 are identical to or different from each other, and each represents a hydrogen atom or the like] in the presence of 0.1 mol or more and 5 mol or less of a base relative to 1 mol of the compound represented by formula (1) to produce the compound represented by formula (3).

## Description

### TECHNICAL FIELD

This application claims the priorities to and the benefits of Japanese Patent Application Nos. 2023-063232 filed on April 10, 2023 and 2023-192831 filed on November 13, 2023, the entire contents of which are incorporated herein by reference.

The present invention relates to methods for producing a 3-hydroxybiphenyl compound and a derivative thereof.

### BACKGROUND ART

Patent Document 1 discloses a useful derivative of a 3-hydroxybiphenyl compound having control efficacy against plant diseases.

Non-Patent Document 1 discloses a method for producing a biphenyl compound having a dialkylamino group or an alkylsulfanyl group at the 3-position.

### CITATION LIST

### PATENT DOCUMENT

Patent Document 1: JP2001-64237A

### NON-PATENT DOCUMENT

Non-Patent Document 1: The Journal of Organic Chemistry, 2009, 74, 7781-7789.

### SUMMARY OF THE INVENTION

### PROBLEMS TO BE SOLVED BY THE INVENTION

An object of the present invention is to provide a novel method for producing a 3-hydroxybiphenyl compound and a novel method for producing a derivative of a 3-hydroxybiphenyl compound using the same.

### MEANS TO SOLVE PROBLEMS

The present inventors have earnestly studied to solve the above problems, and as a result, completed the present invention.

Namely, the present invention provides the followings.
[1] A method for producing a compound represented by formula (3) 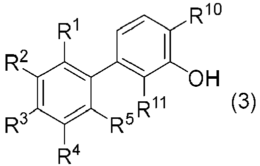 [wherein:
   R¹, R², R³, R⁴, and R⁵ are identical to or different from each other, and each represents a C1-C12 chain hydrocarbon group optionally substituted with one or more fluorine atom(s), a hydrogen atom, a fluorine atom, a chlorine atom, a bromine atom, an iodine atom, a cyano group, or a nitro group; and
   R¹⁰ and R¹¹ are identical to or different from each other, and each represents a hydrogen atom or a C1-C12 chain hydrocarbon group] (hereinafter referred to as "Compound (3)")
   which comprises
   Step 1: reacting a compound represented by formula (1) 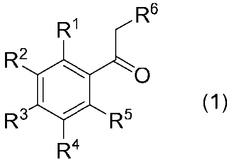 [wherein:
   R¹, R², R³, R⁴, and R⁵ are the same as defined above; and
   R⁶ represents a phenylsulfonyl group, a phenylsulfinyl group, a methanesulfonyl group, a methanesulfinyl group, or a substituent represented by formula (Q-1) 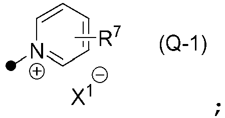
   R⁷ represents a C1-C6 chain hydrocarbon group or a hydrogen atom; and
   X¹ represents a chlorine atom, a bromine atom, or an iodine atom] (hereinafter referred to as "Compound (1)") and a compound represented by formula (2) 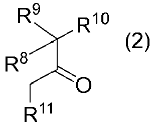 [wherein:
      the combination of R⁸ and R⁹ represents
      a combination wherein R⁸ represents CH₂NR¹²R¹³, CH₂OR¹⁴, or CH₂S(O)ₙR¹⁵, and R⁹ represents a hydrogen atom;
      a combination wherein R⁸ represents a methyl group, and R⁹ represents a chlorine atom, a bromine atom, or an iodine atom; or
      a combination wherein R⁸ and R⁹ are combined with each other to form a methylidene group;
      R¹⁰ and R¹¹ are the same as defined above;
      n represents 0, 1, or 2;
      R¹², R¹³, and R¹⁴ are identical to or different from each other, and each represents a hydrogen atom or a C1-C12 chain hydrocarbon group; and
      R¹⁵ represents a C1-C12 chain hydrocarbon group] (hereinafter referred to as "Compound (2)")
      in the presence of 0.1 mol or more and 5 mol or less of a base relative to 1 mol of the Compound (1) to produce the Compound (3).
[2] The method according to [1], wherein the base is an alkali metal carbonate or an alkali metal hydroxide.
[3] The method according to [1] or [2], wherein R¹⁰ and R¹¹ are identical to or different from each other, and each represents a hydrogen atom or a methyl group.
[4] The method according to any one of [1] to [3], wherein
   R¹ and R⁵ each represents a hydrogen atom; and
   R², R³, and R⁴ are identical to or different from each other, and each represents a hydrogen atom, a fluorine atom, a chlorine atom, a bromine atom, or an iodine atom.
[5] The method according to [4], wherein
   R¹⁰ represents a methyl group; and
   R¹¹ represents a hydrogen atom.
[6] The method according to [5], wherein R², R³, and R⁴ each represents a hydrogen atom.
[7] The method according to [4], wherein
   R², R³, R⁴, and R¹⁰ each represents a hydrogen atom; and
   R¹¹ represents a methyl group.
[8] A method for producing a compound represented by formula (5) 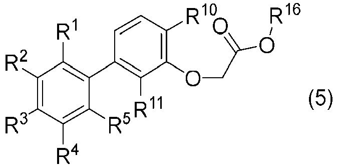 [wherein:
   R¹, R², R³, R⁴, R⁵, R¹⁰, and R¹¹ are the same as defined in any one of [1] and [3] to [7]; and
   R¹⁶ represents a C1-C6 chain hydrocarbon group] (hereinafter referred to as "Compound (5)") which comprises the Step 1 according to any one of [1] to [7];
   and further comprising
   Step 2: reacting the Compound (3) produced in the Step 1 and a compound represented by formula (4) 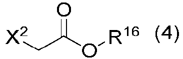 [wherein:
   X² represents a leaving group; and
   R¹⁶ is the same as defined above] (hereinafter referred to as "Compound (4)") in the presence of a base to produce the Compound (5).
[9] A method for producing a compound represented by formula (7) 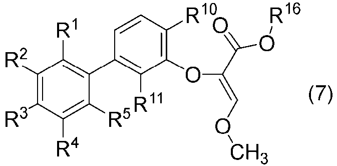 [wherein:
   R¹, R², R³, R⁴, R⁵, R¹⁰, and R¹¹ are the same as defined in any one of [1] and [3] to [7]; and
   R¹⁶ is the same as defined in [8]] (hereinafter referred to as "Compound (7)")
   which comprises the Step 1 and the Step 2 according to [8]; and further comprising
   Step 3: reacting the Compound (5) produced in the Step 2 and a formic acid ester represented by formula (6) 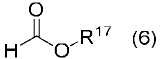 [wherein: R¹⁷ represents a C1-C6 chain hydrocarbon group] (hereinafter referred to as "Compound (6)") in the presence of a base; and
   Step 4: reacting the compound produced in the Step 3 and a methylating agent to produce the Compound (7).
[10] A compound represented by formula (8) 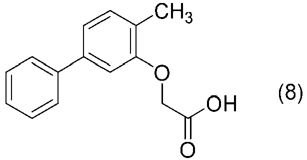 (hereinafter referred to as "Compound (8)").
[11] A compound represented by formula (9) 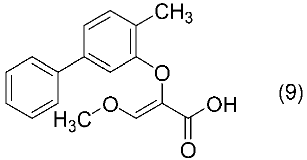 (hereinafter referred to as "Compound (9)").

### EFFECT OF INVENTION

According to the present invention, the Compound (3) can be efficiently produced. Also, the Compound (5) and the Compound (7) can be efficiently produced by using the Compound (3).

### MODE FOR CARRYING OUT THE INVENTION

Hereinafter, the present invention is explained in detail.

The substituents in the present invention are explained as follows.

The expression of "CX-CY" as described herein means that the number of carbon atoms is X to Y. For example, the expression of "C1-C6" means that the number of carbon atoms is 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, a hexyl group, a heptyl group, an octyl group, a decyl group, and a dodecyl group.

Examples of the term of " alkenyl group" include a vinyl group, a 1-propenyl group, a 2-propenyl group, a 1-methyl-1-propenyl group, a 1-methyl-2-propenyl group, a 1,2-dimethyl-1-propenyl group, a 1-ethyl-2-propenyl group, a 3-butenyl group, a 4-pentenyl group, a 5-hexenyl group, a 6-heptenyl group, a 7-octenyl group, a 9-decenyl group, and a 11-dodecenyl group.

Examples of the term of "alkynyl group" include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, a 5-hexynyl group, a 6-heptynyl group, a 7-octynyl group, a 9-decynyl group, and a 11-dodecynyl group.

The Compound (1) is explained as follows.

Examples of the C1-C12 chain hydrocarbon group in R¹, R², R³, R⁴, or R⁵ include the above chain hydrocarbon group having 1 to 12 carbon atom(s). Among them, the above chain hydrocarbon group having 1 to 6 carbon atom(s) is preferable, the above chain hydrocarbon group having 1 to 4 carbon atom(s) is more preferable, and a methyl group, an ethyl group, and a propyl group are still more preferable.

Examples of the C1-C12 chain hydrocarbon group substituted with one or more fluorine atom(s) in R¹, R², R³, R⁴, or R⁵ include chain hydrocarbon group having 1 to 12 carbon atom(s) in which hydrogen atom(s) of the above C1-C12 chain hydrocarbon group is/are substituted with one or more fluorine atom(s). Among them, a trifluoromethyl group, a pentafluoroethyl group, a heptafluoropropyl group, a difluoromethyl group, and a fluoromethyl group are preferable, a trifluoromethyl group, a pentafluoroethyl group, a difluoromethyl group, and a fluoromethyl group are more preferable, a trifluoromethyl group, a difluoromethyl group, and a fluoromethyl group are still more preferable, and a trifluoromethyl group is especially preferable.

Regarding R¹ and R⁵, a hydrogen atom, a fluorine atom, a chlorine atom, a methyl group, and a trifluoromethyl group are preferable, a hydrogen atom and a fluorine atom are more preferable, and a hydrogen atom is still more preferable.

Regarding R², R³, and R⁴, a C1-C6 chain hydrocarbon group optionally substituted with one or more fluorine atom(s), a hydrogen atom, a fluorine atom, a chlorine atom, a bromine atom, and an iodine atom are preferable, a methyl group, an ethyl group, a propyl group, a trifluoromethyl group, a pentafluoroethyl group, a difluoromethyl group, a fluoromethyl group, a hydrogen atom, a fluorine atom, a chlorine atom, a bromine atom, and an iodine atom are more preferable, a methyl group, a trifluoromethyl group, a hydrogen atom, a fluorine atom, a chlorine atom, and a bromine atom are still more preferable, and a hydrogen atom is especially preferable.

In the Compound (1), a compound wherein R⁶ represents a substituent represented by formula (Q-1) specifically refers to a compound represented by formula (10) 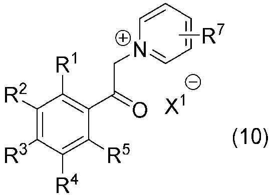 [wherein: R¹, R², R³, R⁴, R⁵, R⁷, and X¹ are the same as defined above].

As R⁶, a phenylsulfonyl group, a phenylsulfinyl group, a methanesulfonyl group, and a methanesulfinyl group are preferable, a methanesulfinyl group and a methanesulfonyl group are more preferable, and a methanesulfonyl group is still more preferable.

Examples of the C1-C6 chain hydrocarbon group in R⁷ include the above chain hydrocarbon group having 1 to 6 carbon atom(s). Among them, the above chain hydrocarbon group having 1 to 4 carbon atom(s) is preferable, and a methyl group, an ethyl group, and a propyl group are more preferable.

The Compound (1) is commercially available or known, or may be produced by using known method(s). For example, see Journal of Organic Chemistry, 1963, 28, 1896-1898., Journal of Organic Chemistry, 1964, 29, 2329-2331., Journal of the American Chemical Society, 1965, 87, 1345-1353., and Ultrasonics Sonochemistry, 2020, 68, 105228.

The Compound (1) has a tautomer (enol isomer), and each isomer and mixtures of isomers at any ratio may be used. Also, when a separately produced Compound (1) is used in the present invention, the Compound (1) may be used without separating it, or may be used as a salt of the Compound (1) (for example, an alkali metal salt such as a lithium salt, a sodium salt, a potassium salt, and a cesium salt).

The Compound (2) is explained as follows.

The Compound (2) wherein R⁸ and R⁹ are combined with each other to form a methylidene group refers to a compound represented by formula (11) 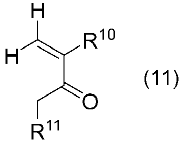 [wherein: R¹⁰ and R¹¹ are the same as defined above] (hereinafter referred to as "Compound (11)").

Examples of the C1-C12 chain hydrocarbon group in R¹⁰ and R¹¹ include the above chain hydrocarbon group having 1 to 12 carbon atom(s). Among them, the above chain hydrocarbon group having 1 to 6 carbon atom(s) is preferable, the above chain hydrocarbon group having 1 to 4 carbon atom(s) is more preferable, and a methyl group, an ethyl group, and a propyl group are still more preferable.

As R¹⁰ and R¹¹, a hydrogen atom, a methyl group, an ethyl group, and a propyl group are preferable, and a combination wherein R¹⁰ represents a methyl group and R¹¹ represents a hydrogen atom is more preferable.

Examples of the C1-C12 chain hydrocarbon group in R¹², R¹³, R¹⁴, and R¹⁵ include the above chain hydrocarbon group having 1 to 12 carbon atom(s). Among them, the above chain hydrocarbon group having 1 to 6 carbon atom(s) is preferable, the above chain hydrocarbon group having 1 to 4 carbon atom(s) is more preferable, a methyl group, an ethyl group, and a propyl group are still more preferable, and a methyl group and an ethyl group are especially preferable.

In the Compound (2), a compound wherein R⁸ represents CH₂NR¹²R¹³ or CH₂OR¹⁴ , and R⁹ represents a hydrogen atom is known or may be produced by using known method(s), or may be produced by reacting the Compound (11) and a corresponding R¹²R¹³NH or R¹⁴OM [wherein: M represents an alkali metal such as sodium and potassium]. Also, in the Compound (2), among a compound wherein R⁸ represents CH₂S(O)ₙR¹⁵ and R⁹ represents a hydrogen atom (hereinafter referred to as "Compound (S)"), a compound wherein n represents 0 or 2 is known or may be produced by using known method(s), or may be produced by reacting the Compound (11) and a corresponding R¹⁵SM [wherein: M is the same as defined above] or R¹⁵S(O)OM [wherein: M is the same as defined above]. Among the Compound (S), a compound wherein n represents 1 is known or may be produced by using known method(s), or may be produced by oxidizing the above Compound (S) wherein n represents 0 according to conventional method(s). A Compound (S) wherein n represents 2 may also be produced by oxidizing a Compound (S) wherein n represents 0 or 1 according to conventional method(s).

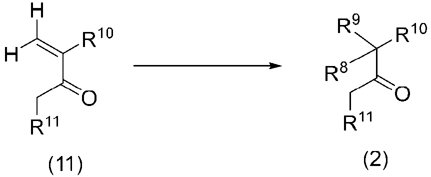

The above reaction for producing the Compound (2) from the Compound (11) is usually carried out in a solvent. Examples of the solvent include hydrocarbon solvents such as heptane, toluene, xylene, and ethylbenzene; halogenated hydrocarbon solvents such as monochlorobenzene; nitrile solvents such as acetonitrile and benzonitrile; ketone solvents such as ethyl methyl ketone; ether solvents such as diisopropyl ether and methyl tert-butyl ether; and mixtures of two or more of them, and hydrocarbon solvents such as heptane, toluene, xylene, and ethylbenzene; halogenated hydrocarbon solvents such as monochlorobenzene; and mixtures of two or more of them are more preferable.

The Compound (11) is commercially available or known, or may be produced by using known method(s).

Among the Compound (2), a compound wherein R⁸ represents a methyl group and R⁹ represents a chlorine atom, a bromine atom, or an iodine atom is commercially available or known, or may be produced by using known method(s).

The Compound (4) is explained as follows.

Examples of the C1-C6 chain hydrocarbon group in R¹⁶ include the above chain hydrocarbon group having 1 to 6 carbon atom(s). Among them, the above chain hydrocarbon group having1 to 3 carbon atom(s) is preferable, a methyl group and an ethyl group are more preferable, and a methyl group is still more preferable.

Examples of the leaving group represented by X² include a chlorine atom, a bromine atom, an iodine atom, a p-toluenesulfonyloxy group, a methanesulfonyloxy group, and a trifluoromethanesulfonyloxy group.

The Compound (4) is commercially available or known, or may be produced by using known method(s).

The Compound (6) is explained as follows.

Examples of the C1-C6 chain hydrocarbon group in R¹⁷ include the above chain hydrocarbon group having 1 to 6 carbon atom(s). Among them, a methyl group, an ethyl group, a propyl group, an isopropyl group, a butyl group, a pentyl group, and a hexyl group are preferable, and a methyl group is more preferable.

The Compound (6) is commercially available or known, or may be produced by using known method(s).

The Step 1 is explained as follows.

In the Step 1, the Compound (1) and the Compound (2) are reacted in the presence of 0.1 mol or more and 5 mol or less of a base relative to 1 mol of the Compound (1) to produce the Compound (3).

When a salt of the above separately produced Compound (1) is used as the Compound (1), the reaction in the Step 1 may be carried out without using the following base or with using a reduced amount of the base.

Examples of the base include inorganic bases, alkali metal alkoxides, organic bases, and mixtures of two or more of them. Examples of the inorganic bases include alkali metal hydroxides such as lithium hydroxide, sodium hydroxide, and potassium hydroxide; alkaline earth metal hydroxides such as calcium hydroxide; alkali metal carbonates such as lithium carbonate, sodium carbonate, potassium carbonate, and cesium carbonate; and alkali metal phosphates such as trisodium phosphate and tripotassium phosphate. Examples of the alkali metal alkoxides include sodium methoxide, sodium ethoxide, sodium-t-butoxide, potassium methoxide, potassium ethoxide, and potassium-t-butoxide. Examples of the organic bases include triethylamine, N,N-diisopropylethylamine, guanidine, and diazabicycloundecene. Among them, alkali metal hydroxides, alkaline earth metal hydroxides, alkali metal carbonates, alkali metal phosphates, alkali metal alkoxides, and mixtures of two or more of them are preferable, alkali metal hydroxides, alkaline earth metal hydroxides, alkali metal carbonates, and mixtures of two or more of them are more preferable, and alkali metal hydroxides, alkali metal carbonates, and mixtures of two or more of them are especially preferable as the base. More specifically, lithium hydroxide, sodium hydroxide, potassium hydroxide, calcium hydroxide, lithium carbonate, sodium carbonate, potassium carbonate, cesium carbonate, and mixtures of two or more of them are more preferable, and sodium hydroxide, potassium carbonate, and mixtures of them are still more preferable as the base. The base may be an anhydride, a hydrate, or a solution, and a commercially available one may be directly used. Examples of solution of the base include an aqueous solution of sodium hydroxide and a methanol solution of sodium methoxide.

The amount of the base to be used is usually 0.1 to 5 mol, preferably 0.5 to 2 mol, more preferably 0.66 to 1.5 mol, and still more preferably 0.8 to 1.2 mol, relative to 1 mol of the Compound (1).

The amount of the Compound (2) to be used is usually 0.5 to 2 mol, preferably 0.66 to 1.5 mol, and more preferably 0.8 to 1.2 mol, relative to 1 mol of the Compound (1).

The reaction is usually carried out in a solvent. As the solvent, a protic solvent and a mixture of protic solvent(s) and aprotic solvent(s) are preferable. Examples of the protic solvent include alcohol solvents such as methanol, ethanol, isopropyl alcohol, and ethylene glycol; water; and mixtures of two or more of them, and methanol, ethylene glycol, water, and mixture of two or more of them are more preferable. Examples of the aprotic solvent to be mixed with the above protic solvent include hydrocarbon solvents such as heptane, toluene, xylene, and ethylbenzene; halogenated hydrocarbon solvents such as monochlorobenzene; nitrile solvents such as acetonitrile and benzonitrile; ketone solvents such as ethyl methyl ketone; ether solvents such as diisopropyl ether and methyl tert-butyl ether; and mixtures of two or more of them. The amount of the aprotic solvent to be used relative to 1 part by weight of the protic solvent is usually 4 parts by weight or less, 1 part by weight or less is preferable, 0.7 part by weight or less is more preferable, and 0.5 part by weight or less is still more preferable.

The amount of the solvent to be used is usually 1 to 20 part(s) by weight relative to 1 part by weight of the Compound (1).

The reaction is carried out by mixing the Compound (1), the Compound (2) and a base, as well as a solvent if needed. In mixing the Compound (1), the Compound (2), and a base, as well as a solvent used if needed, the mixing order is not specifically limited. For example, the reaction may be carried out according to a method wherein a solvent, the Compound (1), and the Compound (2) are mixed, then a base is added thereto; a method wherein a solvent, the Compound (1), and a base are mixed, then the Compound (2) is added thereto; a method wherein the Compound (1) and a solvent are mixed, then the Compound (2) and a base are added thereto; a method wherein the Compound (2) and a solvent are mixed, and then the Compound (1) and a base are added thereto; a method wherein a base and a solvent are mixed, and then the Compound (1) and the Compound (2) are added thereto; a method wherein to a solvent are added the Compound (1), the Compound (2), and a base; or the like. The Compound (1), the Compound (2), and a base may be used after they are mixed with a part of a solvent in advance.

In any of the above methods, regarding each component added to the reaction system, i.e., the Compound (1), the Compound (2), and a base, as well as a solvent used if needed, each component may be added in its whole amount at once, or each component may be divided and added in multiple portions, or each component may be added dropwise. Also, two or more of the components to be added may be added at the same time in their whole amounts at once, or two or more components may be added at the same time and each component may be divided and added in multiple portions, or two or more components may be added at the same time and each component may be added dropwise. Alternatively, two or more of the components to be added may be mixed in advance and then added. Regarding each component to be mixed in advance, each whole amount thereof may be mixed in advance, or a portion thereof may be mixed in advance. Alternatively, two or more components may be mixed in advance to prepare a combination and two or more such combinations with different components may be prepared.

The reaction temperature is usually within a range of 25 to 140°C, preferably 60 to 120°C, and more preferably 70 to 100°C.

The reaction time depends on conditions such as reaction temperature, and is usually 0.1 to 100 hour(s), and preferably 1 to 24 hour(s).

The Compound (3) may be purified according to conventional method(s). For example, when solids are precipitated, the solids produced after completion of the reaction may be collected by filtration to purify the Compound (3). Also, for example, after completion of the reaction, the reaction mixture may be mixed with an acidic aqueous solution such as dilute hydrochloric acid and dilute sulfuric acid or water, subjected to extraction with organic solvent(s), and then the resulting organic layer may be washed, dried, and/or concentrated under reduced pressure to purify the Compound (3). The solvent to be used in the extraction is not specifically limited as long as it can dissolve the Compound (3), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; halogenated hydrocarbon solvents such as monochlorobenzene; ketone solvents such as ethyl methyl ketone; and mixtures of two or more of them. Also, for example, after completion of the reaction, a solvent immiscible with water and a base may be added thereto if needed to carry out acid-base extraction and extract the Compound (3) as a salt into an aqueous layer, then the resulting aqueous layer and an acidic aqueous solution such as dilute hydrochloric acid and dilute sulfuric acid may be mixed, the resulting mixture may be subjected to extraction with organic solvent(s), and then the resulting organic layer may be washed, dried, and/or concentrated under reduced pressure to purify the Compound (3). Examples of the solvent immiscible with water include ether solvents such as diethyl ether, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; halogenated hydrocarbon solvents such as monochlorobenzene; and mixtures of two or more of them. Also, the Compound (3) may be further purified by column chromatography, recrystallization, or the like.

Examples of the solvent which may be used in the recrystallization include a solvent comprising at least one selected from the group consisting of: aromatic hydrocarbon solvents such as toluene, xylene, and ethylbenzene; aliphatic hydrocarbon solvents such as hexane and heptane; halogenated hydrocarbon solvents such as monochlorobenzene; nitrile solvents such as acetonitrile and benzonitrile; ketone solvents such as ethyl methyl ketone and acetone; ether solvents such as 1,4-dioxane, tetrahydrofuran, 2-methyltetrahydrofuran, ethylene glycol dimethyl ether, and t-butyl methyl ether; ester solvents such as ethyl acetate and butyl acetate; alcohol solvents such as methanol and ethanol; water; and aprotic polar solvents such as N,N-dimethylformamide, N-methylpyrrolidone, dimethyl sulfoxide, and sulfolane. Among them, a mixed solvent of at least one selected from the group consisting of aromatic hydrocarbon solvents, halogenated hydrocarbon solvents, nitrile solvents, ketone solvents, ether solvents, ester solvents, and aprotic polar solvents, and at least one selected from the group consisting of aliphatic hydrocarbon solvents, alcohol solvents, and water is preferable.

Also, after completion of the reaction, the Compound (3) may not be isolated to be used in the Step 2.

The Compound (3) may also be purified as a salt. Examples of the salt include alkali metal salts and alkaline earth metal salts. To an organic layer containing the Compound (3) obtained after a work-up such as extraction may be added a base, and the resulting solids may be collected by filtration to purify a salt of the Compound (3). Examples of the base include alkali metal hydroxides such as sodium hydroxide and potassium hydroxide; alkali metal alkoxides such as sodium methoxide, sodium ethoxide, potassium methoxide, and potassium ethoxide; and alkaline earth metal hydroxides such as calcium hydroxide. The base may be an anhydride, a hydrate, or a solution, and a commercially available one may be directly used. Examples of solution of the base include an aqueous solution of sodium hydroxide and a methanol solution of sodium methoxide.

Also, the purified salt of the Compound (3) may also be used in the Step 2.

The Step 2 is explained as follows.

In the Step 2, the Compound (3) produced in the Step 1 and the Compound (4) are reacted in the presence of a base to produce the Compound (5).

When the Compound (3) produced in the Step 1 is used, said Compound (3) to be used may be one produced by the above purification of the Step 1, or may be a mixture comprising the Compound (3) produced by a work-up such as extraction of the reaction mixture after completion of the reaction of the Step 1, or may be a salt of the Compound (3). Examples of the mixture comprising the Compound (3) include the above organic layer containing the Compound (3). When a salt of the Compound (3) is used, the reaction in the Step 2 may be carried out without using the following base or with using a reduced amount of the base.

Examples of the base include inorganic bases, alkali metal alkoxides, organic bases, and mixtures of two or more of them. Examples of the inorganic bases include alkali metal carbonates such as lithium carbonate, sodium carbonate, potassium carbonate, and cesium carbonate; and alkali metal phosphates such as trisodium phosphate and tripotassium phosphate. Examples of the alkali metal alkoxides include NaOR¹⁶ and KOR¹⁶ [wherein: R¹⁶ is the same as defined above]. Examples of the organic bases include triethylamine and diazabicycloundecene. Among them, alkali metal carbonates, alkali metal phosphates, alkali metal alkoxides, and mixtures of two or more of them are preferable as the base. More specifically, lithium carbonate, sodium carbonate, potassium carbonate, cesium carbonate, NaOR¹⁶, KOR¹⁶, and mixtures of two or more of them are more preferable as the base. The base may be an anhydride, a hydrate, or a solution, and a commercially available one may be directly used. Examples of solution of the base include a methanol solution of sodium methoxide.

When an alkali metal alkoxide is used as the base, the reaction may be carried out with heating the reaction mixture to the boiling point of the solvent or higher under ordinary pressure or reduced pressure to distil away an alcohol (R¹⁶OH) produced in the reaction, thereby the reaction may be carried out more efficiently.

The amount of the base to be used is usually 0.5 to 2 mol, preferably 0.66 to 1.5 mol, and more preferably 0.8 to 1.2 mol, relative to 1 mol of the Compound (3).

The amount of the Compound (4) to be used is usually 0.5 to 3 mol, preferably 0.66 to 2.5 mol, and more preferably 0.8 to 1.8 mol, relative to 1 mol of the Compound (3).

The reaction is usually carried out in a solvent. Examples of the solvent include hydrocarbon solvents such as heptane, toluene, xylene, and ethylbenzene; halogenated hydrocarbon solvents such as monochlorobenzene; nitrile solvents such as acetonitrile and benzonitrile; ketone solvents such as ethyl methyl ketone; ether solvents such as diisopropyl ether and methyl tert-butyl ether; and mixtures of two or more of them, hydrocarbon solvents such as heptane, toluene, xylene, and ethylbenzene; halogenated hydrocarbon solvents such as monochlorobenzene; and mixtures of two or more of them are more preferable, and xylene, ethylbenzene, and mixtures of them are especially preferable.

The amount of the solvent to be used is usually 1 to 20 part(s) by weight relative to 1 part by weight of the Compound (3).

The reaction is carried out by mixing the Compound (3), the Compound (4), and a base, as well as a solvent if needed.

The reaction temperature is usually within a range of -20 to 140°C, preferably 0 to 120°C, and more preferably 60 to 100°C.

The reaction time depends on conditions such as reaction temperature, and is usually 0.1 to 100 hour(s), and preferably 1 to 24 hour(s).

The Compound (5) may be purified according to conventional method(s). For example, when solids are precipitated, the solids produced after completion of the reaction may be collected by filtration to purify the Compound (5). Also, for example, after completion of the reaction, the reaction mixture may be mixed with an acidic aqueous solution such as dilute hydrochloric acid and dilute sulfuric acid or water, subjected to extraction with organic solvent(s), and then the resulting organic layer may be washed, dried, and/or concentrated under reduced pressure to purify the Compound (5). The solvent to be used in the extraction is not specifically limited as long as it can dissolve the Compound (5), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (5) may be further purified by column chromatography, recrystallization, or the like.

Examples of the solvent which may be used in the recrystallization include a solvent comprising at least one selected from the group consisting of: aromatic hydrocarbon solvents such as toluene, xylene, and ethylbenzene; aliphatic hydrocarbon solvents such as hexane and heptane; halogenated hydrocarbon solvents such as monochlorobenzene; nitrile solvents such as acetonitrile and benzonitrile; ketone solvents such as ethyl methyl ketone and acetone; ether solvents such as 1,4-dioxane, tetrahydrofuran, 2-methyltetrahydrofuran, ethylene glycol dimethyl ether, and t-butyl methyl ether; ester solvents such as ethyl acetate and butyl acetate; alcohol solvents such as methanol and ethanol; water; and aprotic polar solvents such as N,N-dimethylformamide, N-methylpyrrolidone, dimethyl sulfoxide, and sulfolane. Among them, a mixed solvent of at least one selected from the group consisting of aromatic hydrocarbon solvents, halogenated hydrocarbon solvents, nitrile solvents, ketone solvents, ether solvents, ester solvents, and aprotic polar solvents, and at least one selected from the group consisting of aliphatic hydrocarbon solvents, alcohol solvents, and water is preferable.

Also, after completion of the reaction, the Compound (5) may not be isolated to be used in the Step 3.

The Step 3 is explained as follows.

In the Step 3, the Compound (5) produced in the Step 2 and the Compound (6) are reacted in the presence of a base.

According to the reaction of the Step 3, a compound represented by formula (12) 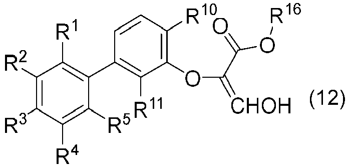 [wherein: R¹, R², R³, R⁴ R⁵, R¹⁰, R¹¹ , and R¹⁶ are the same as defined above] (hereinafter referred to as "Compound (12)") may be produced.

When the Compound (5) produced in the Step 2 is used, said Compound (5) to be used may be one produced by the above purification of the Step 2, or may be a mixture comprising the Compound (5) produced by a work-up such as extraction of the reaction mixture after completion of the reaction of the Step 2.

Examples of the base include alkali metal hydrides such as sodium hydride and potassium hydride; alkali metal alkoxides such as sodium methoxide, sodium ethoxide, potassium methoxide, potassium ethoxide, and potassium-t-butoxide; alkali metal amides such as sodium amide, lithium amide, lithium diisopropylamide, sodium hexamethyldisilazide, and lithium hexamethyldisilazide; and mixtures of two or more of them.

The amount of the base to be used is usually a ratio of 1 to 10 mol relative to 1 mol of the Compound (5).

The amount of the Compound (6) to be used is usually a ratio of 1 to 10 mol relative to 1 mol of the Compound (5).

The reaction is usually carried out in a solvent. Examples of the solvent include ether solvents such as 1,4-dioxane, tetrahydrofuran, ethylene glycol dimethyl ether, and t-butyl methyl ether; aliphatic hydrocarbon solvents such as hexane and heptane; aromatic hydrocarbon solvents such as toluene, xylene, and ethylbenzene; halogenated hydrocarbon solvents such as monochlorobenzene; organic bases such as pyridine, triethylamine, and N,N-dimethylaniline; nitrile solvents such as acetonitrile; aprotic polar solvents such as N,N-dimethylformamide, dimethyl sulfoxide, N,N-dimethyl-2-imidazolidinone, and sulfolane; and mixtures of two or more of them.

The reaction temperature is usually within a range of 0 to 80°C.

The reaction time is usually within a range of 1 to 48 hour(s).

The Compound (12) may be purified according to conventional method(s). For example, when solids are precipitated, the solids produced after completion of the reaction may be collected by filtration to purify the Compound (12). Also, for example, after completion of the reaction, the reaction mixture may be mixed with an acidic aqueous solution such as dilute hydrochloric acid and dilute sulfuric acid, subjected to extraction with organic solvent(s), and then the resulting organic layer may be washed, dried, and/or concentrated under reduced pressure to purify the Compound (12). The solvent to be used in the extraction is not specifically limited as long as it can dissolve the Compound (12), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (12) may be further purified by column chromatography, recrystallization, or the like.

Also, after completion of the reaction, the Compound (12) may not be isolated to be used in the Step 4, or may not be subjected to a work-up such as extraction to be used in the Step 4.

Also, the Compound (12) may be reacted with a base such as an alkali metal hydroxide to prepare a solution of a salt of the Compound (12) (for example, an alkali metal salt such as a sodium salt) in a protic solvent such as water to be used in the Step 4.

The Step 4 is explained as follows.

In the Step 4, the compound produced in the Step 3 and a methylating agent are reacted to produce the Compound (7).

The compound produced in the Step 3, i.e., the Compound (12) to be used in the Step 4 may be one produced by the work-up such as extraction or the purification in the Step 3, may be a mixture comprising the Compound (12) produced by the work-up such as extraction of the reaction mixture if needed after completion of the reaction of the Step 3 without neutralizing it, or may be a mixture comprising the Compound (12) produced by partially neutralizing or completely neutralizing the reaction mixture after completion of the reaction of Step 3. When a mixture comprising the Compound (12) produced by not neutralizing or by partially neutralizing the reaction mixture after completion of the reaction of the Step 3 is used in the Step 4, the Compound (12) forms a alkali metal salt such as a lithium salt, a sodium salt, and a potassium salt, and thus the reaction of the Step 4 may be carried out without using the following base which may be used with a methylating agent such as dimethyl sulfate, or with using a reduced amount of the base. Examples of the acid used in the above partial neutralization or complete neutralization include inorganic acids such as hydrochloric acid, sulfuric acid, and phosphoric acid; and organic acids such as acetic acid, propionic acid, and benzoic acid.

The reaction is usually carried out in a solvent. Examples of the solvent which may be used include a solvent comprising at least one selected from the group consisting of: aromatic hydrocarbon solvents, aliphatic hydrocarbon solvents, halogenated hydrocarbon solvents, ether solvents, ketone solvents, ester solvents, alcohol solvents, water, nitrile solvents, and aprotic polar solvents. Such solvent may be one selected from the group consisting of aromatic hydrocarbon solvents, aliphatic hydrocarbon solvents, halogenated hydrocarbon solvents, ether solvents, ketone solvents, ester solvents, alcohol solvents, water, nitrile solvents, and aprotic polar solvents, may be a mixed solvent of two or more selected from the group consisting of aromatic hydrocarbon solvents, aliphatic hydrocarbon solvents, halogenated hydrocarbon solvents, ether solvents, ketone solvents, ester solvents, alcohol solvents, water, nitrile solvents, and aprotic polar solvents, or may be a mixed solvent of at least one selected from the group consisting of aromatic hydrocarbon solvents, aliphatic hydrocarbon solvents, halogenated hydrocarbon solvents, ether solvents, ketone solvents, ester solvents, alcohol solvents, water, nitrile solvents, and aprotic polar solvents, and a compound other than them.

Examples of the aromatic hydrocarbon solvents include toluene, xylene, and ethylbenzene. Examples of the aliphatic hydrocarbon solvents include hexane and heptane. Examples of the halogenated hydrocarbon solvents include monochlorobenzene. Examples of the ether solvents include 1,4-dioxane, tetrahydrofuran, ethylene glycol dimethyl ether, and t-butyl methyl ether. Examples of the ketone solvents include acetone and ethyl methyl ketone. Examples of the ester solvents include butyl acetate and ethyl acetate. Examples of the alcohol solvents include methanol and ethanol. Examples of the nitrile solvents include acetonitrile and benzonitrile. Examples of the aprotic polar solvents include N,N-dimethylformamide, N-methylpyrrolidone, dimethyl sulfoxide, and sulfolane.

Examples of the methylating agent include a compound represented by CH₃-X³ [wherein: X³ represents a leaving group], dimethyl sulfate, dimethyl carbonate, and diazo compounds. Among them, dimethyl sulfate is preferable. Examples of the leaving group represented by X³ include a chlorine atom, a bromine atom, an iodine atom, a p-toluenesulfonyloxy group, a methanesulfonyloxy group, and a trifluoromethanesulfonyloxy group. Examples of the diazo compounds include trialkylsilyldiazomethane such as trimethylsilyldiazomethane; and diazomethane.

The amount of the methylating agent to be used is usually 1 to 5 mol relative to 1 mol of the Compound (12). When a mixture comprising the Compound (12) produced in the Step 3 is used as the Compound (12), the amount of the methylating agent to be used is usually 1 to 5 mol relative to 1 mol of the Compound (5) in the Step 3.

When a compound represented by CH₃-X³, dimethyl sulfate, or dimethyl carbonate is used as the methylating agent, it is preferable to carry out the reaction in the presence of a base. Examples of the base include inorganic bases, alkali metal alkoxides, and mixtures of two or more of them. Examples of the inorganic bases include sodium hydroxide, potassium hydroxide, sodium carbonate, potassium carbonate, calcium carbonate, and sodium hydride. Examples of the alkali metal alkoxides include potassium-t-butoxide, sodium methoxide, potassium methoxide, and sodium ethoxide.

When a base is used, the amount of the base to be used is preferably 1 to 10 mol relative to 1 mol of the Compound (12).

When a diazo compound is used as the methylating agent, it is preferable to use a protic solvent. Examples of the protic solvent include the above alcohol solvents.

The reaction may be carried out in the presence of a catalyst. Examples of the catalyst include quaternary ammonium salts such as tetrabutylammonium chloride, tetrabutylammonium bromide, benzyltrimethylammonium chloride, benzyltrimethylammonium bromide, benzyltriethylammonium chloride, and benzyltriethylammonium bromide. When a catalyst is used, the amount of the catalyst to be used is usually 0.005 to 0.2 mol relative to 1 mol of the Compound (12).

The reaction temperature is usually within a range of -20 to 100°C.

The reaction time is usually within a range of 1 to 48 hour(s).

The Compound (7) may be purified according to conventional method(s). For example, when solids are precipitated, the solids produced after completion of the reaction may be collected by filtration to purify the Compound (7). Also, for example, after completion of the reaction, the reaction mixture may be mixed with an acidic aqueous solution such as dilute hydrochloric acid and dilute sulfuric acid or water, subjected to extraction with organic solvent(s), and then the resulting organic layer may be washed, dried, and/or concentrated under reduced pressure to purify the Compound (7). The solvent to be used in the extraction is not specifically limited as long as it can dissolve the Compound (7), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (7) may be further purified by column chromatography, recrystallization, or the like.

The reaction is carried out by mixing the Compound (12) and a methylating agent, and if needed, a solvent, a base, and a catalyst. The mixing order of the Compound (12) and a methylating agent, and if needed, a solvent, a base, and, a catalyst is not specifically limited. For example, the Compound (12), a solvent, and a methylating agent may be mixed at one time, the Compound (12) and a solvent may be mixed, then a methylating agent may be added thereto, or a methylating agent and a solvent may be mixed, and then the Compound (12) may be added thereto. When the Compound (12) or a methylating agent is added to the reaction system, the Compound (12) or the methylating agent may be added as a mixture with the above solvent.

The Compound (8) is explained as follows.

The Compound (8) is produced by hydrolyzing the ester of a compound represented by formula (13) 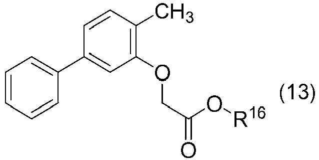 [wherein: R¹⁶ is the same as defined above] (hereinafter referred to as "Compound (13)"). The Compound (13) is the Compound (5) wherein R¹, R², R³, R⁴, and R⁵ each represents a hydrogen atom, R¹⁰ represents a methyl group, and R¹¹ represents a hydrogen atom.

The reaction in which the ester of the Compound (13) is hydrolyzed to produce the Compound (8) is explained as follows. The Compound (13) and water are reacted in the presence of an acid to produce the Compound (8).

Examples of the acid include hydrochloric acid, sulfuric acid, and p-toluenesulfonic acid.

The amount of the acid to be used is usually 0.01 to 1 mol relative to 1 mol of the Compound (13).

The reaction is usually carried out in a solvent. As the solvent, one which does not easily react with the acid is preferable, and examples thereof include hydrocarbon solvents such as heptane, toluene, xylene, and ethylbenzene; halogenated hydrocarbon solvents such as monochlorobenzene; and mixtures of two or more of them.

The amount of the solvent to be used is usually 1 to 20 part(s) by weight relative to 1 part by weight of the Compound (13).

The amount of water to be used is usually 1 to 100 mol relative to 1 mol of the Compound (13). Water may be used as a solvent.

The reaction temperature is usually within a range of 0 to 140°C, preferably 20 to 100°C, and more preferably 40 to 80°C.

The reaction time depends on conditions such as reaction temperature, and is usually 0.1 to 100 hour(s), and preferably 1 to 24 hour(s).

The Compound (8) may be purified according to conventional method(s). For example, when solids are precipitated, the solids produced after completion of the reaction may be collected by filtration to purify the Compound (8). Also, for example, after completion of the reaction, the reaction mixture may be mixed with an acidic aqueous solution such as dilute hydrochloric acid and dilute sulfuric acid or water, subjected to extraction with organic solvent(s), and then the resulting organic layer may be washed, dried, and/or concentrated under reduced pressure to purify the Compound (8). The solvent to be used in the extraction is not specifically limited as long as it can dissolve the Compound (8), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (8) may be further purified by column chromatography, recrystallization, or the like.

Also, the Compound (13) and water may be reacted in the presence of a base to produce the Compound (8).

Examples of the base include inorganic bases, organic bases, and mixtures of two or more of them. Examples of the inorganic bases include alkali metal hydroxides such as lithium hydroxide, sodium hydroxide, and potassium hydroxide; alkaline earth metal hydroxides such as calcium hydroxide; alkali metal carbonates such as lithium carbonate, sodium carbonate, potassium carbonate, and cesium carbonate; and alkali metal phosphates such as trisodium phosphate and tripotassium phosphate. Examples of the organic bases include triethylamine and diazabicycloundecene.

The amount of the base to be used is usually 1 to 10 mol relative to 1 mol of the Compound (13).

The reaction is usually carried out in a solvent. Examples of the solvent include hydrocarbon solvents such as heptane, toluene, xylene, and ethylbenzene; halogenated hydrocarbon solvents such as monochlorobenzene; ether solvents such as 1,4-dioxane, tetrahydrofuran, ethylene glycol dimethyl ether, and t-butyl methyl ether; and mixtures of two or more of them.

The amount of the solvent to be used is usually 1 to 20 part(s) by weight relative to 1 part by weight of the Compound (13).

The amount of water to be used is usually 1 to 100 mol relative to 1 mol of the Compound (13). Water may be used as a solvent. When the reaction is carried out using an alkali metal hydroxide and an alkaline earth metal hydroxides as a base, the reaction may be carried out without using water.

The reaction temperature is usually within a range of 0 to 140°C, preferably 20 to 100°C, and more preferably 40 to 80°C.

The reaction time depends on conditions such as reaction temperature, and is usually 0.1 to 100 hour(s), and preferably 1 to 24 hour(s).

The Compound (8) may be purified according to conventional method(s). For example, after completion of the reaction, the reaction mixture may be mixed with an acidic aqueous solution such as dilute hydrochloric acid and dilute sulfuric acid, subjected to extraction with organic solvent(s), and then the resulting organic layer may be washed, dried, and/or concentrated under reduced pressure to purify the Compound (8). The solvent to be used in the extraction is not specifically limited as long as it can dissolve the Compound (8), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (8) may be further purified by column chromatography, recrystallization, or the like.

Also, in the Step 2, when the Compound (5) is the Compound (13), the Compound (8) may be produced during the reaction, and contained in the reaction mixture.

The carboxy group of the Compound (8) may be esterified to be derivatized into the Compound (13). Specifically, for example, the Compound (8) and the alcohol (R¹⁰OH) may be subjected to dehydration-condensation to produce the Compound (13).

In this regard, in the Step 2, the derivatization of the Compound (8) which may be contained in the reaction mixture after completion of the reaction into the starting material, the Compound (13) means the yield improvement of the Compound (13), i.e., the corresponding Compound (5). Namely, the Compound (8) can be deemed as a production intermediate of the corresponding Compound (5).

The reaction in which the Compound (8) and the alcohol (R¹⁶OH) are subjected to dehydration-condensation to produce the Compound (13) is explained as follows.

The Compound (8) and the alcohol (R¹⁶OH) are reacted in the presence of an acid to produce the Compound (13), i.e., the Compound (5) wherein R¹, R², R³, R⁴, and R⁵ each represents a hydrogen atom, R¹⁰ represents a methyl group, and R¹¹ represents a hydrogen atom.

Examples of the acid include sulfuric acid and p-toluenesulfonic acid.

The amount of the acid to be used is usually 0.01 to 1 mol relative to 1 mol of the Compound (8).

The reaction is usually carried out in a solvent. As the solvent, one which does not easily react with the acid is preferable, and examples thereof include hydrocarbon solvents such as heptane, toluene, xylene, and ethylbenzene; halogenated hydrocarbon solvents such as monochlorobenzene; and mixtures of two or more of them.

The amount of the solvent to be used is usually 1 to 20 part(s) by weight relative to 1 part by weight of the Compound (8).

The amount of the alcohol (R¹⁶OH) to be used is usually 1 to 100 mol relative to 1 mol of the Compound (8). The above alcohol may be used as a solvent.

The reaction is carried out by mixing the Compound (8), the alcohol (R¹⁶OH), an acid, and a solvent.

The reaction temperature is usually within a range of 0 to 140°C, preferably 60 to 130°C, and more preferably 80 to 120°C.

The reaction time depends on conditions such as reaction temperature, and is usually 0.1 to 100 hour(s), and preferably 1 to 24 hour(s).

The Compound (13) may be purified according to conventional method(s). For example, when solids are precipitated, the solids produced after completion of the reaction may be collected by filtration to purify the Compound (13). Also, for example, after completion of the reaction, the reaction mixture is mixed with water, subjected to extraction with organic solvent(s), and then the resulting organic layer may be washed, dried, and/or concentrated under reduced pressure to purify the Compound (13). The solvent to be used in the extraction is not specifically limited as long as it can dissolve the Compound (13), and examples thereof include ether solvents such as diethyl ether, tetrahydrofuran, tert-butyl methyl ether, and cyclopentyl methyl ether; hydrocarbon solvents such as pentane, hexane, heptane, octane, benzene, toluene, xylene, ethylbenzene, mesitylene, cyclohexane, and cyclopentane; ketone solvents such as ethyl methyl ketone; halogenated hydrocarbon solvents such as monochlorobenzene; and mixtures of two or more of them. Also, the Compound (13) may be further purified by column chromatography, recrystallization, or the like.

Also, the Compound (13) wherein R¹⁶ represents a methyl group, i.e., a compound represented by formula (14) 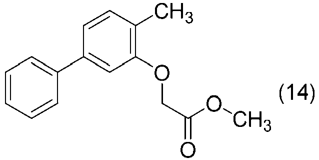 (hereinafter referred to as "Compound (14)") may also be produced by reacting the Compound (8) and a methylating agent. In this regard, the Compound (14) is the Compound (5) wherein R¹, R², R³, R⁴, and R⁵ each represents a hydrogen atom, R¹⁰ represents a methyl group, R¹¹ represents a hydrogen atom, and R¹⁶ represents a methyl group.

The reaction in which the Compound (8) and a methylating agent are reacted to produce the Compound (14) is explained as follows. The reaction, purification, and the like may be carried out according to the Step 4 using the Compound (8) instead of the compound produced in the Step 3.

When the Compound (5) is the Compound (14) in the Step 2, the reaction of the Compound (8) and a methylating agent may also be carried out using the Compound (8) contained in the reaction mixture after completion of the reaction of the Step 2. In this case, the corresponding Compound (5), i.e., the Compound (14) is newly produced depending on the amount of the Compound (8) contained in the reaction mixture after completion of the reaction of the Step 2. This means that the yield of the Compound (14) increases.

The Compound (9) is explained as follows.

The Compound (9) is produced by hydrolyzing the ester of a compound represented by formula (15) 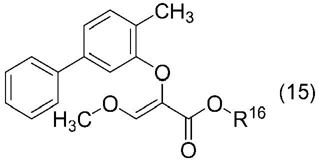 [wherein: R¹⁶ is the same as defined above] (hereinafter referred to as "Compound (15)"). The Compound (15) is the Compound (7) wherein R¹, R², R³, R⁴, and R⁵ each represents a hydrogen atom, R¹⁰ represents a methyl group, and R¹¹ represents a hydrogen atom.

The reaction in which the ester of the Compound (15) is hydrolyzed to produce the Compound (9) is explained as follows. The Compound (15) and water are reacted in the presence of a base to produce the Compound (9).

The reaction, purification, and the like may be carried out using the Compound (15) instead of the Compound (13) according to the reaction in which the Compound (13) and water are reacted in the presence of a base to produce the Compound (8).

Also, in the Step 4, when the Compound (7) is the Compound (15), the Compound (9) may be produced during the reaction, and contained in the reaction mixture.

The carboxy group of the Compound (9) may be esterified to be derivatized into the Compound (15). Specifically, for example, the Compound (9) and the alcohol (R¹⁶OH) may be subjected to dehydration-condensation to produce the Compound (15).

In this regard, in the Step 4, the derivatization of the Compound (9) which may be contained in the reaction mixture after completion of the reaction into the starting material, the Compound (15) means the yield improvement of the Compound (15), i.e., the corresponding Compound (7). Namely, the Compound (9) can be deemed as a production intermediate of the corresponding Compound (7).

The reaction in which the Compound (9) and the alcohol (R¹⁶OH) are subjected to dehydration-condensation to produce the Compound (15) is explained as follows.

The Compound (9) and the alcohol (R¹⁶OH) are reacted in the presence of an acid to produce the Compound (15), i.e., the Compound (5) wherein R¹, R², R³, R⁴, and R⁵ each represents a hydrogen atom, R¹⁰ represents a methyl group, and R¹¹ represents a hydrogen atom.

The reaction, purification, and the like may be carried out using the Compound (9) instead of the Compound (8) according to the reaction in which the Compound (8) and the alcohol (R¹⁶OH) are subjected to dehydration-condensation to produce the Compound (13).

Also, the Compound (15) wherein R¹⁶ represents a methyl group, i.e., a compound represented by formula (16) 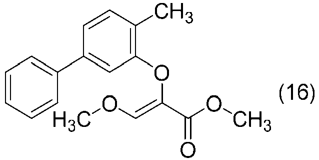 (hereinafter referred to as "Compound (16)") may also be produced by reacting the Compound (9) and a methylating agent. In this regard, the Compound (16) is the Compound (7) wherein R¹, R², R³, R⁴, and R⁵ each represents a hydrogen atom, R¹⁰ represents a methyl group, R¹¹ represents a hydrogen atom, and R¹⁶ represents a methyl group.

The reaction in which the Compound (9) and a methylating agent are reacted to produce the Compound (16) is explained as follows.

The reaction, purification, and the like may be carried out according to the Step 4 using the Compound (9) instead of the compound produced in the Step 3.

When the Compound (7) is the Compound (16) in the Step 4, the reaction of the Compound (9) and a methylating agent may also be carried out using the Compound (9) contained in the reaction mixture after completion of the reaction of the Step 4. In this case, the corresponding Compound (7), i.e., the Compound (16) is newly produced depending on the amount of the Compound (9) contained in the reaction mixture after completion of the reaction of the Step 4. This means that the yield of the Compound (16) increases.

### EXAMPLES

Hereinafter, the present invention is illustrated more in detail by Examples, but the present invention is not limited to the following Examples.

The Compound (A) is the Compound (1) wherein R¹, R², R³, R⁴, and R⁵ each represents a hydrogen atom, and R⁶ represents a methanesulfonyl group. The Compound (B) is the Compound (1) wherein R¹, R², R³, R⁴, and R⁵ each represents a hydrogen atom, and R⁶ represents a phenylsulfonyl group. The Compound (K) is the Compound (1) wherein R¹, R², R³, R⁴, and R⁵ each represents a hydrogen atom, and R⁶ represents a methanesulfinyl group. The Compound (C) is the Compound (2) wherein R⁸ represents CH₂NR¹²R¹³, R⁹ and R¹¹ each represents a hydrogen atom, and R¹⁰, R¹², and R¹³ each represents a methyl group. The Compound (D) is the Compound (2) wherein R⁸ represents CH₂NR¹²R¹³, R⁹, R¹¹, and R¹² each represents a hydrogen atom, R¹⁰ represents a methyl group, and R¹³ represents an ethyl group. The Compound (E) is the Compound (2) wherein R⁸ represents CH₂NR¹²R¹³, R⁹, R¹¹, and R¹² each represents a hydrogen atom, and R¹⁰ and R¹³ each represents a methyl group. The Compound (F) is the Compound (2) wherein R⁶ represents CH₂S(O)ₙR¹⁵, R⁹ and R¹¹ each represents a hydrogen atom, R¹⁰ represents a methyl group, R¹⁵ represents an ethyl group, and n represents 0. The Compound (G) is the Compound (2) wherein R⁸ represents CH₂OR¹⁴, R⁹ and R¹¹ each represents a hydrogen atom, and R¹⁰ and R¹⁴ each represents a methyl group. The Compound (H) is the Compound (2) wherein R⁸ and R⁹ are combined with each other to form a methylidene group, R¹⁰ represents a methyl group, and R¹¹ represents a hydrogen atom. The Compound (L) is the Compound (2) wherein R⁶ represents CH₂NR¹²R¹³, R⁹ and R¹⁰ each represents a hydrogen atom, and R¹¹, R¹², and R¹³ each represents a methyl group. The Compound (I) is the Compound (3) wherein R¹, R², R³, R⁴, R⁵, and R¹¹ each represents a hydrogen atom, and R¹⁰ represents a methyl group. The Compound (J) is the Compound (12) wherein R¹, R², R³, R⁴, R⁵, and R¹¹ each represents a hydrogen atom, and R¹⁰ and R¹⁶ each represents a methyl group. The Compound (M) is the Compound (3) wherein R¹, R², R³, R⁴, R⁵, and R¹⁰ each represents a hydrogen atom, and R¹¹ represents a methyl group. In the following Compound (B), Ph represents a phenyl group, in the Compound (A), the Compound (K), the Compound (C), the Compound (D), the Compound (E), the Compound (F), the Compound (G), the Compound (H), the Compound (L), and the Compound (M), Me represents a methyl group, and in the Compound (D) and the Compound (F), Et represents an ethyl group.

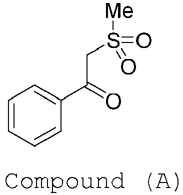

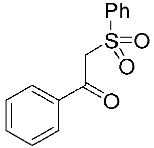

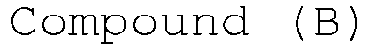

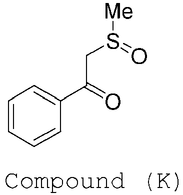

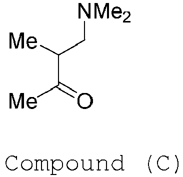

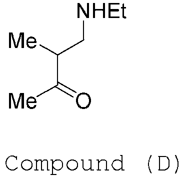

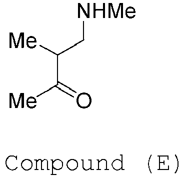

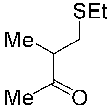

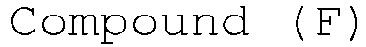

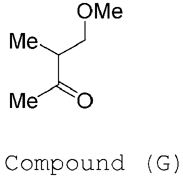

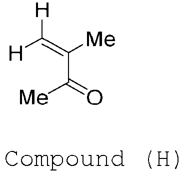

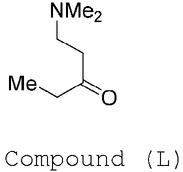

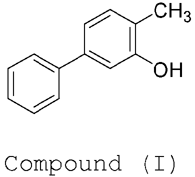

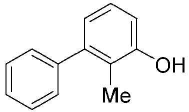

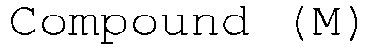

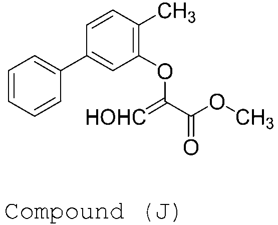

In the following Examples, quantitative analysis was carried out using high performance liquid chromatography, unless otherwise described. The yield of the target compound was calculated using absolute calibration curve method from the peak area of the target compound. The analysis conditions are as follows.

### [Analysis conditions of high performance liquid chromatography]

Mobile phase: Solution A: 5 mM aqueous solution of ammonium carbonate, Solution B: acetonitrile
Column: SUMIPAX (registered trademark) ODS Z-CLUE, particle size: 3 µm, 4.6 mm I.D. × 100 mm
UV measurement wavelength: 238 nm
Flow rate: 1.0 mL/min
Column oven: 30°C
Pump: two LC-20AD (manufactured by Shimadzu Corporation)
(high-pressure gradient)
Gradient conditions: sending a solution with the concentration gradient described in Table LC1.

**Table LC1**

| Time (min) | Solution A (%) | Solution B (%) |
|---|---|---|
| 0 | 65 | 35 |
| 35 | 20 | 80 |
| 50 | 20 | 80 |

In the following Examples, the reaction temperature means the outside temperature of the reaction container, unless otherwise described.

### Example 1 (example of Step 1)

The Compound (A) (99.4 mg), the Compound (C) (64.7 mg), a 48% by weight aqueous solution of sodium hydroxide (40.8 mg), and water (1 mL) were mixed, and stirred at 80 to 100°C for 5 hours. The resulting mixture was analyzed by high performance liquid chromatography to confirm that the Compound (I) was produced at a yield of 75%.

### Example 2 (example of Step 1)

The Compound (A) (99.8 mg), the Compound (C) (66.8 mg), a 48% by weight aqueous solution of sodium hydroxide (42.0 mg), and ethylene glycol (1 mL) were mixed, and stirred at 80 to 100°C for 5 hours. The resulting mixture was analyzed by high performance liquid chromatography to confirm that the Compound (I) was produced at a yield of 73%.

### Example 3 (example of Step 1)

The Compound (A) (98.8 mg), the Compound (D) (65.5 mg), a 48% by weight aqueous solution of sodium hydroxide (44.5 mg), and water (0.5 mL) were mixed, and stirred at 80 to 100°C for 5 hours. The resulting mixture was analyzed by high performance liquid chromatography to confirm that the Compound (I) was produced at a yield of 62%.

### Example 4 (example of Step 1)

The Compound (A) (99.1 mg), the Compound (E) (67.2 mg), a 48% by weight aqueous solution of sodium hydroxide (43.3 mg), and water (1 mL) were mixed, and stirred at 80 to 100°C for 5 hours. The resulting mixture was analyzed by high performance liquid chromatography to confirm that the Compound (I) was produced at a yield of 70%.

### Example 5 (example of Step 1)

The Compound (A) (99.1 mg), the Compound (F) (79.2 mg), potassium carbonate (145.0 mg), and ethylene glycol (0.5 mL) were mixed, and stirred at 80 to 100°C for 5 hours. The resulting mixture was analyzed by high performance liquid chromatography to confirm that the Compound (I) was produced at a yield of 61%.

### Example 6 (example of Step 1)

The Compound (A) (99.1 mg), the Compound (G) (59.1 mg), a 48% by weight aqueous solution of sodium hydroxide (41.7 mg), and ethylene glycol (1 mL) were mixed, and stirred at 80 to 100°C for 5 hours. The resulting mixture was analyzed by high performance liquid chromatography to confirm that the Compound (I) was produced at a yield of 64%.

### Example 7 (example of Step 1)

The Compound (A) (98.3 mg), the Compound (H) (43.1 mg), potassium carbonate (68.5 mg), and ethylene glycol (0.5 mL) were mixed, and stirred at 80 to 100°C for 5 hours. The resulting mixture was analyzed by high performance liquid chromatography to confirm that the Compound (I) was produced at a yield of 72%.

### Example 8 (example of Step 1)

The Compound (A) (100.5 mg), the Compound (H) (42.2 mg), a 48% by weight aqueous solution of sodium hydroxide (43.8 mg), and water (0.5 mL) were mixed, and stirred at 80 to 100°C for 5 hours. The resulting mixture was analyzed by high performance liquid chromatography to confirm that the Compound (I) was produced at a yield of 63%.

### Example 9 (example of Step 1)

The Compound (B) (131.4 mg), the Compound (H) (42.7 mg), a 48% by weight aqueous solution of sodium hydroxide (44.2 mg), and ethylene glycol (0.5 mL) were mixed, and stirred at 80 to 100°C for 5 hours. The resulting mixture was analyzed by high performance liquid chromatography to confirm that the Compound (I) was produced at a yield of 56%.

### Example 10 (example of Step 1)

The Compound (K) (101.0 mg), the Compound (F) (85.1 mg), sodium methoxide (33.5 mg), and water (1.0 mL) were mixed, and stirred at 80 to 100°C for 5 hours. The resulting mixture was analyzed by high performance liquid chromatography to confirm that the Compound (I) was produced at a yield of 72%.

### Example 11 (example of Step 1)

The Compound (K) (103.0 mg), the Compound (F) (87.6 mg), a 48% by weight aqueous solution of sodium hydroxide (48.0 mg), and water (1.0 mL) were mixed, and stirred at 80 to 100°C for 5 hours. The resulting mixture was analyzed by high performance liquid chromatography to confirm that the Compound (I) was produced at a yield of 72%.

### Example 12 (example of Step 1)

The Compound (K) (104.0 mg), the Compound (G) (70.3 mg), a 48% by weight aqueous solution of sodium hydroxide (49.0 mg), and methanol (1.0 mL) were mixed, and stirred at 80 to 100°C for 5 hours. The resulting mixture was analyzed by high performance liquid chromatography to confirm that the Compound (I) was produced at a yield of 62%.

### Example 13 (example of Step 1)

The Compound (K) (102.0 mg), the Compound (F) (89.2 mg), potassium carbonate (81.2 mg), and methanol (1.0 mL) were mixed, and stirred at 80 to 100°C for 5 hours. The resulting mixture was analyzed by high performance liquid chromatography to confirm that the Compound (I) was produced at a yield of 66%.

### Example 14 (example of Step 1)

The Compound (K) (103.0 mg), the Compound (G) (69.6 mg), a 48% by weight aqueous solution of sodium hydroxide (50.9 mg), and ethylene glycol (1.0 mL) were mixed, and stirred at 80 to 100°C for 5 hours. The resulting mixture was analyzed by high performance liquid chromatography to confirm that the Compound (I) was produced at a yield of 63%.

### Example 15 (example of Step 1)

The Compound (K) (101.0 mg), the Compound (C) (76.6 mg), potassium carbonate (81.2 mg), and ethylene glycol (1.0 mL) were mixed, and stirred at 80 to 100°C for 5 hours. The resulting mixture was analyzed by high performance liquid chromatography to confirm that the Compound (I) was produced at a yield of 60%.

### Example 16 (example of Step 1)

The Compound (A) (100.0 mg), the Compound (E) (59.3 mg), a 48% by weight aqueous solution of sodium hydroxide (54.6 mg), xylene (0.8 mL), water (0.1 mL), and methanol (0.1 mL) were mixed, and stirred at 80 to 100°C for 5 hours. The resulting mixture was analyzed by high performance liquid chromatography to confirm that the Compound (I) was produced at a yield of 53%.

### Example 17 (example of Step 1)

The Compound (A) (45.0 mg), the Compound (H) (19.9 mg), a 48% by weight aqueous solution of sodium hydroxide (25.0 mg), xylene (0.5 mL), water (0.25 mL), and methanol (0.25 mL) were mixed, and stirred at 80 to 100°C for 5 hours. The resulting mixture was analyzed by high performance liquid chromatography to confirm that the Compound (I) was produced at a yield of 51%.

### Example 18 (example of Step 1)

The Compound (A) (101.0 mg), the Compound (E) (58.7 mg), a 48% by weight aqueous solution of sodium hydroxide (42.5 mg), xylene (0.5 mL), and water (0.5 mL) were mixed, and stirred at 80 to 100°C for 5 hours. The resulting mixture was analyzed by high performance liquid chromatography to confirm that the Compound (I) was produced at a yield of 64%.

### Example 19 (example of Step 1)

The Compound (A) (102.0 mg), the Compound (H) (45.0 mg), a 48% by weight aqueous solution of sodium hydroxide (47.0 mg), xylene (0.33 mL), water (0.33 mL), and methanol (0.33 mL) were mixed, and stirred at 80 to 100°C for 5 hours. The resulting mixture was analyzed by high performance liquid chromatography to confirm that the Compound (I) was produced at a yield of 55%.

### Example 20 (example of Step 1)

The Compound (A) (101.0 mg), the Compound (C) (66.7 mg), a 48% by weight aqueous solution of sodium hydroxide (44.4 mg), xylene (0.33 mL), and water (0.66mL) were mixed, and stirred at 80 to 100°C for 5 hours. The resulting mixture was analyzed by high performance liquid chromatography to confirm that the Compound (I) was produced at a yield of 53%.

### Example 21 (example of Step 1)

The Compound (A) (102.0 mg), the Compound (F) (75.2 mg), a 48% by weight aqueous solution of sodium hydroxide (47.9 mg), xylene (0.33 mL), and water (0.66 mL) were mixed, and stirred at 80 to 100°C for 5 hours. The resulting mixture was analyzed by high performance liquid chromatography to confirm that the Compound (I) was produced at a yield of 50%.

### Example 22 (example of Step 1)

The Compound (A) (103.0 mg), the Compound (L) (69.1 mg), potassium carbonate (71.1 mg), and ethylene glycol (1.0 mL) were mixed, and stirred at 80 to 100°C for 5 hours. The resulting mixture was analyzed by high performance liquid chromatography to confirm that the Compound (M) was produced at a yield of 53%.

### Example 23 (example of Step 2)

To a mixture of a xylene solution (140.0 g) comprising 32.7% by weight of the Compound (I), xylene (73.4 g), and methyl chloroacetate (44.1 g) was added dropwise a 28% by weight solution of sodium methoxide in methanol (73.0 g) at 80°C, dimethyl sulfate (3.4 g) was added thereto, and the resulting mixture was stirred for 1 hour. After the mixture was allowed to cool to 60°C, water (126.5 g) was added thereto to separate the resulting solution, and the resulting organic layer was concentrated. To the resulting concentrated residue were added methanol and water to precipitate crystals, and the Compound (14) was isolated as crystals (59.7 g).

### Example 24 (example of Step 3)

Sodium methoxide (21.91 g) was suspended in xylene (50.43 g) and heated to 30°C, to the resulting mixture was added dropwise a solution of mixture of the Compound (14) (50.1 g), xylene (150.1 g), and methyl formate (23.68 g) over 2 hours, and the resulting mixture was stirred at 30°C for 2.5 hours. The resulting mixture was cooled to 0°C, water (150.4 g), 35% by weight hydrochloric acid (10.14 g), and a 27% by weight aqueous solution of sodium hydroxide (1.11 g) were sequentially added thereto, the resulting mixture was stirred, then allowed to stand, and separated. The resulting aqueous layer (250.5 g) was analyzed by high performance liquid chromatography to confirm that the yield of sodium salt of the Compound (J), i.e., the Compound (12) wherein R¹, R², R³, R⁴, and R⁵ each represents a hydrogen atom, R¹⁰ represents a methyl group, R¹¹ represents a hydrogen atom, and R¹⁶ represents a methyl group was 91.2% on the basis of the Compound (14).

### Example 25 (example of Step 4)

To the aqueous layer (4.47 g) produced in Example 24 were added benzonitrile (3.91 g), tetrabutylammonium bromide (0.04 g), a 27% by weight aqueous solution of sodium hydroxide (0.11 g), and dimethyl sulfate (0.62 g), and the resulting mixture was stirred at 40°C for 2 hours. The resulting mixture was analyzed by high performance liquid chromatography to confirm that the yield of the Compound (16) was 92.8% on the basis of the sodium salt of the Compound (J).

### Example 26 (synthesis of Compound (8))

To a mixture of the Compound (14) (1.00 g) and tetrahydrofuran (10 mL) was added a 48% by weight aqueous solution of sodium hydroxide (660 mg), and the resulting mixture was stirred at 25°C for 5 hours. The mixture was diluted with water, and then hydrochloric acid was added thereto to adjust the pH to 1 or less. The resulting solution was concentrated, and the concentrated mixture was filtered to isolate the Compound (8) as solids (0.83 g).

The values of ¹H-NMR of the Compound (8) are shown below.
¹H-NMR (DMSO-D6) δ: 7.64-7.62 (2H, m), 7.45-7.44 (2H, m), 7.35-7.33 (1H, m), 7.23 (1H, d), 7.15 (1H, dd), 7.07 (1H, d), 4.82 (2H, s), 2.22 (3H, s).

### Example 27 (synthesis of Compound (14))

To a mixture of the Compound (8) (1.00 g), N,N-dimethylformamide (10 mL), and potassium carbonate (860 mg) was added dimethyl sulfate (810 mg), and the resulting mixture was stirred at 25°C for 1 hour. The mixture was diluted with water, then hydrochloric acid was added thereto to adjust the pH to 1 or less, and the resulting mixture was filtered to isolate the Compound (14) as solids (0.95 g).

### Example 28 (synthesis of Compound (9))

The Compound (16) (8.0 g), tetrahydrofuran (40.0 g), and a 48% by weight aqueous solution of sodium hydroxide (8.94 g) were mixed, and stirred at 65°C for 3 hours. To the resulting mixture was added sulfuric acid to adjust the pH to 2 or less, and the resulting solvent was concentrated. The resulting concentrated residue was subjected to extraction with chloroform, the resulting solution was dried over sodium sulfate, then hexane was added thereto to precipitate crystals, and the Compound (9) (5.6 g) was isolated as crystals.

The values of ¹H-NMR of the Compound (9) are shown below.
¹H-NMR (CDCl₃) δ:7.51-7.48 (2H, m), 7.43 (1H, s), 7.41-7.37 (2H, m), 7.33-7.28 (1H, m), 7.21 (1H, d), 7.14 (1H, dd), 6.93 (1H, d), 3.89 (3H, s), 2.37 (3H, s).

### Example 29 (synthesis of Compound (16))

To a mixture of the Compound (9) (0.52 g), N,N-dimethylformamide (5.2 mL), and potassium carbonate (0.39 g) was added dimethyl sulfate (0.36 g), and the resulting mixture was stirred at 25°C for 1 hour. The mixture was diluted with water, then hydrochloric acid was added thereto to adjust the pH to 1 or less, and the resulting mixture was filtered to isolate the Compound (16) as solids (0.44 g).

The retention time in the analysis by high performance liquid chromatography of each compound described in the above Examples is shown in the following table.

| Compound | Retention time (min) |
|---|---|
| Compound (I) | 19.11 |
| Compound (M) | 18.53 |
| Compound (14) | 26.13 |
| Compound (J) | 3.84 |
| Compound (16) | 25.56 |
| the Compound (8) | 2.37 |
| the Compound (9) | 2.31 |

### INDUSTRIAL APPLICABILITY

According to the present invention, the Compound (3) can be efficiently produced. Also, the Compound (5) and the Compound (7) can be efficiently produced by using the Compound (3).

## Claims

1. A method for producing a compound represented by formula (3) 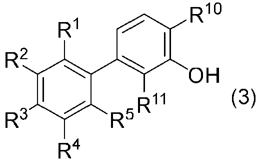 [wherein:
R¹, R², R³, R⁴, and R⁵ are identical to or different from each other, and each represents a C1-C12 chain hydrocarbon group optionally substituted with one or more fluorine atom(s), a hydrogen atom, a fluorine atom, a chlorine atom, a bromine atom, an iodine atom, a cyano group, or a nitro group; and
R¹⁰ and R¹¹ are identical to or different from each other, and each represents a hydrogen atom or a C1-C12 chain hydrocarbon group]
which comprises
Step 1: reacting a compound represented by formula (1) 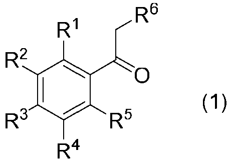 [wherein:
R¹, R², R³, R⁴, and R⁵ are the same as defined above;
R⁶ represents a phenylsulfonyl group, a phenylsulfinyl group, a methanesulfonyl group, a methanesulfinyl group, or a substituent represented by formula (Q-1) 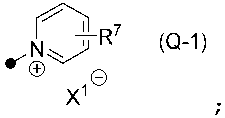
R⁷ represents a C1-C6 chain hydrocarbon group or a hydrogen atom; and
X¹ represents a chlorine atom, a bromine atom, or an iodine atom]
and a compound represented by formula (2) 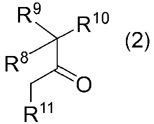 [wherein:
the combination of R⁸ and R⁹ represents
a combination wherein R⁸ represents CH₂NR¹²R¹³, CH₂OR¹⁴, or CH₂S(O)ₙR¹⁵, and R⁹ represents a hydrogen atom;
a combination wherein R⁸ represents a methyl group, and R⁹ represents a chlorine atom, a bromine atom, or an iodine atom; or
a combination wherein R⁸ and R⁹ are combined with each other to form a methylidene group;
R¹⁰ and R¹¹ are the same as defined above;
n represents 0, 1, or 2;
R¹², R¹³, and R¹⁴ are identical to or different from each other, and each represents a hydrogen atom or a C1-C12 chain hydrocarbon group; and
R¹⁵ represents a C1-C12 chain hydrocarbon group] in the presence of 0.1 mol or more and 5 mol or less of a base relative to 1 mol of the compound represented by formula (1) to produce the compound represented by formula (3).

2. The method according to claim 1, wherein the base is an alkali metal carbonate or an alkali metal hydroxide.

3. The method according to claim 1 or 2, wherein R¹⁰ and R¹¹ are identical to or different from each other, and each represents a hydrogen atom or a methyl group.

4. The method according to any one of claims 1 to 3,
wherein
R¹ and R⁵ each represents a hydrogen atom; and
R², R³, and R⁴ are identical to or different from each other, and each represents a hydrogen atom, a fluorine atom, a chlorine atom, a bromine atom, or an iodine atom.

5. The method according to claim 4, wherein
R¹⁰ represents a methyl group; and
R¹¹ represents a hydrogen atom.

6. The method according to claim 5, wherein R², R³, and R⁴ each represents a hydrogen atom.

7. The method according to claim 4, wherein
R², R³, R⁴, and R¹⁰ each represents a hydrogen atom; and
R¹¹ represents a methyl group.

8. A method for producing a compound represented by formula (5) 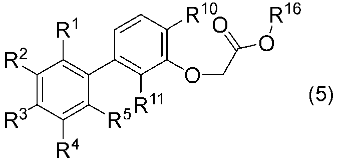 [wherein:
R¹, R², R³, R⁴, R⁵, R¹⁰, and R¹¹ are the same as defined in any one of claims 1 and 3 to 7; and
R¹⁶ represents a C1-C6 chain hydrocarbon group] which comprises the Step 1 according to any one of claims 1 to 7;
and further comprising
Step 2: reacting the compound represented by formula (3) produced in the Step 1 and a compound represented by formula (4) 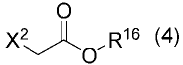 [wherein:
X² represents a leaving group; and
R¹⁶ is the same as defined above]
in the presence of a base to produce the compound represented by formula (5).

9. A method for producing a compound represented by formula (7) 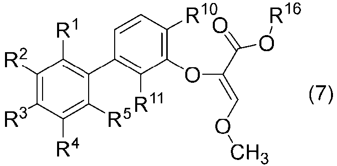 [wherein:
R¹, R², R³, R⁴, R⁵, R¹⁰, and R¹¹ are the same as defined in any one of claims 1 and 3 to 7; and
R¹⁶ is the same as defined in claim 8] which comprises the Step 1 and the Step 2 according to claim 8;
and further comprising
Step 3: reacting the compound represented by formula (5) produced in the Step 2 and a formic acid ester represented by formula (6) 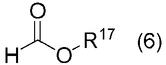 [wherein: R¹⁷ represents a C1-C6 chain hydrocarbon group] in the presence of a base; and
Step 4: reacting the compound produced in the Step 3 and a methylating agent to produce the compound represented by formula (7).

10. A compound represented by formula (8)

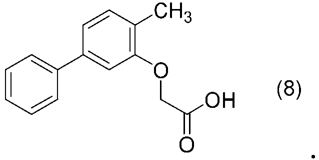

11. A compound represented by formula (9)
